# EUROPÄISCHE PATENTSCHRIFT

(11) **EP 3 096 808 B1**
(45) Veröffentlichungstag und Bekanntmachung des Hinweises auf die Patenterteilung: **13.03.2024**
(21) Anmeldenummer: 15700731.1
(22) Anmeldetag: 21.01.2015
(51) Int. Cl.: A61M 60/113, A61M 60/232, A61M 60/457, A61M 60/531, A61M 60/554, A61M 1/36

(54) **VORRICHTUNG ZUR REGELUNG UND VORGABE DER PUMPRATE VON BLUTPUMPEN**
DEVICE FOR REGULATING AND SPECIFYING THE PUMP RATE OF BLOOD PUMPS
DISPOSITIF DE RÉGULATION ET DE DÉFINITION DU DÉBIT DE POMPES D'ASSISTANCE CIRCULATOIRE

(30) Priorität: 22.01.2014 DE 102014000678
(43) Veröffentlichungstag der Anmeldung: 30.11.2016
(73) Patentinhaber: Fresenius Medical Care Deutschland GmbH, 61352 Bad Homburg (DE)
(72) Erfinder: HEIDE, Alexander, 65817 Eppstein (DE); PETERS, Arne, 61352 Bad Homburg (DE); WEIS, Manfred, 66606 St. Wendel (DE); WIKTOR, Christoph, 63571 Gelnhausen (DE)
(74) Vertreter: Hitzelberger, Christoph Michael
(86) Internationale Anmeldenummer: PCT/EP2015/051045
(87) Internationale Veröffentlichungsnummer: WO 2015/110437

(56) Entgegenhaltungen:
- EP-A1- 0 513 421
- EP-A2- 2 517 740
- US-A- 4 610 656
- US-A- 5 965 089
- US-A1- 2003 045 772
- US-A1- 2012 150 089
- US-A1- 2013 126 404

## Beschreibung

### Technisches Gebiet

Die Erfindung betrifft das Gebiet der Regelung von Blutpumpen in medizintechnischen Geräten, siehe z.B. US 2012/150089 A1, US 5 965 089 A, EP 0 513 421 A1, US 4 610 656 A, EP 2 517 740 A2, US 2003/045772 A1 and US 2013/126404 A1, insbesondere in Blutbehandlungsgeräten wie Dialysegeräte.

### Hintergrund

Blutbehandlungsgeräte sind medizintechnische Geräte zur Behandlung des Bluts eines Patienten, die in der Regel extrakorporal durchgeführt wird. Beispielsweise können dem Blut Medikamente zugesetzt oder Blutbestandteile entzogen werden, oder es kann erwärmt oder gekühlt werden. Zu diesem Zweck wird das Blut oftmals außerhalb des Körpers mit einer Blutpumpe gepumpt, behandelt und dem Körper des Patienten wieder zurückgegeben. Ein solcher Blutkreislauf wird als extrakorporaler Blutkreislauf bezeichnet.

Im Folgenden soll die Erfindung anhand des Beispiels Dialysegerät als Ausführungsform eines medizintechnischen Geräts zur extrakorporalen Blutbehandlung erläutert werden.

Weitere Blutbehandlungsgeräte sind beispielsweise Geräte zur Unterstützung der Herz-Lungentätigkeit, wie Blutoxygenatoren, oder Geräte zur Unterstützung der Leberfunktion, die Bluttoxine durch Adsorption aus dem Blut entfernen. Oftmals kombinieren Geräte zur Unterstützung der Leberfunktion Dialyseverfahren und Adsorptionsverfahren in einem Gerät, wie beispielsweise das unter dem Markennamen Prometheus vertriebene Gerät der Anmelderin.

Dialysegeräte sind Blutbehandlungsgeräte, bei denen Blut eines Patienten über eine Blutleitung einer Blutbehandlungskomponente zugeführt, durch die Blutbehandlungskomponente behandelt und über die Fluidleitung, die in einen arteriellen und einen venösen Blutzweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin, deren Inhalt hiermit vollumfänglich im Offenbarungsgehalt der vorliegenden Anmeldung einbezogen wird.

Die Dialyse ist ein Verfahren zur Blutreinigung von Patienten mit akuter oder chronischer Niereninsuffizienz. Grundsätzlich unterscheidet man hierbei zwischen Verfahren mit einem extrakorporalen Blutkreislauf, wie der Hämodialyse, der Hämofiltration oder der Hämodiafiltration und der Peritonealdialyse, bei der kein extrakorporaler Blutkreislauf vorgesehen ist.

Das Blut wird bei der Hämodialyse in einem extrakorporalen Kreislauf durch die Blutkammer eines Dialysators, der die Blutbehandlungskomponente ausbildet, geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysier-flüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Verfahren der Hämodialyse, der Hämofiltration und der Hämodiafiltration werden in der Regel mit automatischen Hämodialysegeräten durchgeführt, wie sie beispielsweise von der Anmelderin unter der Bezeichnung 5008 vertrieben werden.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Geräte zur extrakorporalen Blutbehandlung wie Dialysegeräte, haben umfangreiche Funktionen. Um diese Funktionen zu steuern, sind Geräte zur extrakorporalen Blutbehandlung mit zumindest einer Steuervorrichtung ausgerüstet. Diese können als CPU (central processing unit) oder Mikrokontroller ausgeführt sein, die von Softwareprogrammen programmiert werden.

Zur Förderung von Flüssigkeiten in Geräten zur extrakorporalen Blutbehandlung werden Pumpen unterschiedlicher Ausgestaltung verwendet. Für den extrakorpora-len Blutkreislauf werden oftmals peristaltische Schlauchrollenpumpen verwendet. Diese Schlauchrollenpumpen finden häufig in der Medizintechnik Anwendung, da mit ihnen ein kontaktloser Transport einer Flüssigkeit möglich ist. Darüber hinaus liefern sie theoretisch einen zur Drehzahl proportionalen Fluss in weiten Bereichen unabhängig von den Flusswiderständen stromauf und stromab der Pumpe. Bei einer Blutpumpe in extrakorporalen Behandlungsverfahren bezeichnet man die zuführende (Saug-) Seite als arterielle Seite mit einem sich einstellenden Unterdruck von typisch ca. -100 bis -300 mmHG (Quecksilbersäule gegenüber Außendruck), und die abführende Seite als venöse Seite mit einem sich einstellenden Überdruck gegenüber dem Außendruck.

Die DE 3 326 785A1 zeigt eine typische Ausgestaltung für eine solche okkludierende Schlauchrollenpumpe, wonach das Fördermedium mittels eines periodisch okkludierten Schlauches bewegt wird.

Von der Grundkonzeption her weist eine Rollenpumpe einen Stator und einen Rotor auf. Der Stator ist an dem Pumpengehäuse ausgebildet und weist eine Vertiefung auf, an deren stetig verlaufender senkrechter Wand ein Pumpenschlauch anliegt. Der Bereich, an dem der Pumpenschlauch an der Wand anliegt, bildet das Pumpen-bett, das die Kontur eines Kreisausschnittes hat.

Durch den Mittelpunkt dieses Kreisausschnittes verläuft die Drehachse eines Rotors, der an seinen freien Enden drehbar gelagerte Rollen aufweist. Bei Drehung des Rotors in Arbeitsrichtung gelangen die Rollen mit dem Pumpenschlauch, der an der Kreiskontur des Pumpenbettes anliegt, in Berührung und drücken ihn bei weiterlaufender Drehung so weit zusammen, dass er flüssigkeitsdicht (okkludierend) abschließt.

Durch ein weiteres Abrollen der Rollen auf dem Pumpenschlauch wird das im Pumpenschlauch befindliche Fördermedium weiter transportiert. In der Mehrzahl der Fälle weist eine derartige Rollenpumpe zwei Rollen auf, die so an dem Rotor angebracht sind, dass die Verbindungslinie durch ihre Drehachsen am Rotor durch die Drehachse des Rotors verläuft.

Andere Pumpentypen, die in Geräten zur extrakorporalen Blutbehandlung zum Einsatz kommen können, sind beispielsweise Zentrifugalpumpen, Membranpumpen oder Zahnradpumpen.

Der Pumpentyp ist maßgeblich für die Beanspruchung des zu fördernden Mediums. Dies ist insbesondere bei einem extrakorporalen Blutkreislauf von Bedeutung, denn durch das Pumpen kann das Blut geschädigt werden, insbesondere kann es zur Zerstörung der Erythrozyten, also der roten Blutzellen kommen (Hämolyse). Dies kann insbesondere mechanisch, beispielsweise durch die Quetschung innerhalb eines Blutschlauchs, als auch durch zu hohe Drücke oder Druckgradienten geschehen.

Charakteristisch für eine Schlauchrollenpumpe ist der pulsatile, nicht stetige Fluss, der durch den fortwährenden Eingriff der Rollen in das Pumpenschlauchsegment hervorgerufen wird. Beim Eingriff der Rollen in das Schlauchsegment wird der Schlauch zusammengequetscht und dabei Flüssigkeit verdrängt. Diese Flüssigkeit wird sowohl in als auch gegen die Flussrichtung verdrängt. Stromaufwärts der Rolle überlagert sich die verdrängte Flüssigkeit im laufenden Betrieb mit dem Fluss in Richtung Pumpe und führt somit zu einer kurzzeitigen Netto-Verringerung des Flusses, wodurch der arterielle Druck weniger negativ wird, solange bis der Schlauch komplett okkludiert ist. Danach beschleunigt sich die Flüssigkeit im Schlauch wieder, und der arterielle Druck sinkt wieder. Stromabwärts der Schlauchrollenpumpe entsteht ein plötzlicher Druckabfall sobald die Rolle aus dem Pumpensegment austritt, und es zu einem Druckausgleich zwischen dem Unterdruck im bislang eingeschlossenen Segment zwischen den Rollen und dem Überdruck stromabwärts der Pumpe kommt.

Dabei können Druckspitzen (bzw. Flussspitzen) im Bereich der Punktionsstelle der Nadel, die das extrakorporale Blut einem Patienten zurückgibt, auftreten und Scherkräfte hervorrufen, welche im Extremfall zur Thrombosierung (Gerinselbildung) an den Gefäßwänden und zu Hämolyse, also zur Zerstörung von Erythrozyten (rote Blutkörperchen), führen können. Stromaufwärts der Pumpe kann es beim Ausgleich zwischen Hoch- und Niederdrucksystem ebenfalls zu hohen Scherkräften kommen.

Ein anderer in Geräten zur extrakorporalen Blutbehandlung eingesetzter Pumpentyp ist die Impeller- oder Zentrifugalpumpe. Zentrifugalpumpen enthalten im Wesentlichen ein Gehäuse zur Aufnahme eines Flügelrades. Dieses Flügelrad kann in einer Ausführungsform durch eine durch das Gehäuse verlaufende Achse gedreht werden. Eine alternative Ausführungsform sieht vor, dass das Flügelrad kontaktlos gedreht wird. Hierbei ist das Flügelrad beispielsweise mit einem Magneten fest verbunden und kann über ein von außen anliegendes rotierendes Magnetfeld, welches beispielsweise durch Feldspulen erzeugt wird, durch magnetische Kopplung zur Rotation angeregt werden. Das rotierende Flügelrad bewegt die im Gehäuse befindliche Flüssigkeit von einem Flüssigkeitseingang zu einem Flüssigkeitsausgang. Durch das Wirkprinzip liefern Zentrifugalpumpen einen konstanten Differenzdruck, wobei der Ausgangsdruck der gepumpten Flüssigkeit vom Eingangsdruck, der Viskosität der Flüssigkeit, der Pumprate und der Drehzahl abhängt. Druckpulse in der geförderten Flüssigkeit wie bei den peristaltischen Pumpen kommen bei Zentrifugalpumpen im Normalbetrieb mit konstanter Drehgeschwindigkeit des Flügelrads nicht vor. Hierdurch wird Hämolyse, die durch pulsatile Blutförderung verursacht wird, reduziert.

Eine gesteigerte Hämolyse kann zu einer ernsthaften Gefährdung des Patienten führen. So gehören neben einer Anämie auch Entzündungen, Belastungen der Leber und ein erhöhtes Thromboserisiko zu den direkten Konsequenzen einer Freisetzung des für den Sauerstofftransport zuständigen Hämoglobins aus den Erythrozyten. Neben Hämoglobin tritt auch das Elektrolyt Kalium aus den geschädigten Zellen. Verstärkte Freisetzung von Kalium stört die Reizweiterleitung in Nerven und Muskeln und kann unter anderem zu akuten Herzproblemen führen.

Unabhängig vom eingesetzten Blutpumpentyp besteht generell bei der Förderung von Blut die Gefahr, Zellbestandteile des Blutes zu schädigen.

### Beschreibung

Es ist daher Aufgabe der vorliegenden Erfindung, eine Vorrichtung und ein Verfahren (nicht beansprucht) zu schaffen, bei denen das Auftreten von Blutschädigungen beim Fördern von Blut vermindert wird.

Die Lösung dieser Aufgaben erfolgt erfindungsgemäß durch eine Vorrichtung mit den Merkmalen des Anspruchs 1.

Vorteilhafte Ausführungsformen sind Gegenstand der Unteransprüche.

In einer Ausführungsform ist vorgesehen, eine neue Pumpenrate einer Blutpumpe bei der Förderung von Blut einzustellen, wenn der Blutdruck stromaufwärts und/oder stromabwärts der Blutpumpe mit einer aktuell vorliegenden Pumprate einen Grenzwert verletzt, der von einem Betriebsparameter oder einem Pumpenparameter der Blutpumpe abhängt. Diese vorgegebene neue Blutpumpenrate würde dazu führen, dass der Grenzwert nicht mehr verletzt wird, bzw. dass die Differenz des Blutdrucks stromaufwärts von diesem Grenzwert und/oder stromabwärts der Blutpumpe von diesem Grenzwert verringert wird. Der Grenzwert ist mit einer Blutschädigungsrate verknüpft. Die Blutpump ist eine Zentrifugalpumpe und der Betriebsparameter ist die Pumprate der Blutpumpe.

Als Betriebsparameter sind für Blutpumpen insbesondere die Pumpenrate, also das geförderte Volumen pro Zeiteinheit, sowie die sich einstellenden Blutdrücke stromaufwärts und stromabwärts der Blutpumpe von besonderer Bedeutung.

Betriebsparameter im Sinne der vorliegenden Erfindung sind allgemein Parameter, die vom Arbeitspunkt der Blutpumpe abhängen und können somit neben der Pumprate, sowie den flussaufwärts oder flussabwärts der Blutpumpe herrschenden Flüssigkeitsdrücken, beispielsweise auch die Leistungsaufnahme des elektrischen Antriebs der Blutpumpe umfassen.

Unter einem Pumpenparameter wird im Sinne der vorliegenden Erfindung ein Parameter verstanden, der von der Ausgestaltung der Blutpumpe abhängt. Ein solcher Pumpenparameter ist beispielsweise die Okklusionskraft einer Schlauchrollenpumpe, also die Kraft, mit der die Rollen einer Schlauchrollenpumpe den einliegenden Schlauch gegen das Pumpbett pressen. Ein Pumpenparameter ist also nicht vom Arbeitspunkt der Blutpumpe abhängig, sondern von deren baulichen Ausgestaltung. Weiterhin ist vorgesehen, dass eine Vorrichtung die so vorgegebene Pumprate einstellt.

Es hat sich gezeigt, dass es bei der Förderung von Blut durch einen extrakorporalen Blutkreislauf unvermeidlich ist, dass bestimmte Scherkräfte auf die Zellen des Blutes einwirken. Übersteigen diese Kräfte ein bestimmtes Maß, werden die Zellen geschädigt. Damit verbunden ist eine Blutschädigungsrate.

Darüber hinaus werden bei starken Unterdrücken, beispielsweise kleiner als -300mmHG oder -400mmHG gegenüber dem Umgebungsdruck, kleinste Luftbläschen vor und in der Pumpe in den Blutstrom eingebracht.

Das geschieht zum einen durch Undichtigkeiten an Konnektions- und Zugabestellen, wie beispielsweise einem Konnektor, über den Antikoagulanzien wie Heparin dem extrakorporalen Blutkreislauf zugeführt werden, und zum anderen durch Entgasungen im Blut.

Diese Mikroblasen können aufgrund ihrer geringen Größe nur schlecht im extrakorporalen Blutkreislauf abgeschieden werden. Gelangen sie in den Patienten, so kann das zum Verschluss von Kapillargefäßen führen, was sich negativ auf das Wohlbefinden des Patienten auswirkt.

Der Erfindung liegt die Erkenntnis zu Grunde, dass für das Auftreten von Blutschädigungen wie Hämolyse beim Fördern von Blut mit Zentrifugalpumpen maßgeblich die Höhe des Blutdrucks stromabwärts und stromaufwärts der Blutpumpe bzw. die Differenz dieser Drücke und die Höhe des Blutflusses verantwortlich ist. Weiterhin beeinflusst je nach Pumpentyp auch die Pumprate, also das geförderter Blutvolumen pro Zeiteinheit, das Auftreten von Blutschädigungen.

Bei Zentrifugalpumpen ist die festzustellende Hämoglobinfreisetzung, die ein Maß für das Auftreten von Hämolyse als Blutschädigung ist, weitgehend unabhängig vom Blutfluss, wird aber bei steigendem Blutdruck größer. Hierbei kann grundsätzlich der Blutdruck stromaufwärts oder stromabwärts der Zentrifugalpumpe bewertet werden, da bei Zentrifugalpumpen die Flüssigkeitsdrücke stromaufwärts und stromabwärts der Pumpe über die Pumprate der Zentrifugalpumpe und einer für die Zentrifugalpumpe charakteristischen Kennlinie miteinander verknüpft sind.

Das ist eine direkte Folge des nicht okkludierenden Pumpprinzips von Zentrifugalpumpen. Somit können sowohl die Höhe des Blutdrucks stromaufwärts und stromabwärts der Zentrifugalpumpe, als auch die Differenz dieser Drücke gleichwertig in Bezug auf die Pumprate gesetzt werden, um eine Aussage über die zu erwartende Blutschädigung zu erhalten.

Schlauchrollenpumpen sind im Normalbetrieb okkludierende Pumpen, wobei zumindest eine Rolle der Pumpe den Schlauch zwischen Pumpeneingang und Pumpenausgang vollständig okkludiert. Daher gibt es keine direkte Abhängigkeit der Flüssigkeitsdrücke stromaufwärts und stromabwärts der Schlauchrollenpumpe, da Pumpeneingang und Pumpenausgang bei Schlauchrollenpumpen im Normalfall voneinander druckdicht isoliert sind.

Maßgeblich für die Druckverhältnisse bei Schlauchrollenpumpen sind die Umdrehungszahl, die weitgehend die Pumprate bestimmt, die Viskosität des geförderten Mediums und die Flusswiderstände der Flusswege stromaufwärts und stromabwärts der Schlauchrollenpumpe. Diese Flusswiderstände werden bei Blutpumpen beispielsweise durch die verwendeten Blutschläuche, Kanülen und Dialysefilter bestimmt.

Dialysefilter können sich während einer Dialysebehandlung zusetzen (sogenanntes Clotting) und das Blut des Patienten kann während der Behandlung durch das Entziehen überschüssigen Plasmawassers dicker werden, so dass sich die Flusswiderstände und die Viskosität des Blutes verändern können, was im Laufe der Behandlung zu veränderten Blutdrücken im extrakorporalen Blutkreislauf führen kann.

So kann es bei stark erhöhtem Flusswiderstand flussabwärts der Schlauchrollen-pumpe zu Spitzen des Blutdrucks kommen, die die Gefahr beinhalten, dass Undichtigkeiten in den angeschlossenen Schlauchstücken oder dem Dialysefilter auftreten, bei dem das Blut des Patienten in den Dialysatkreislauf übertritt oder sogar in die Umwelt gelangt.

Um eine solche Situation zu vermeiden, sind Schlauchrollenpumpen zur Blutförderung normalerweise mit federnd gelagerten Schlauchrollen ausgestattet. Übersteigt die durch den Blutdruck erzeugte Kraft auf die Schlauchrollen die Federkraft, mit der die Rolle gegen den Schlauch gepresst wird, wird diese radial zur Umdrehungsrichtung zur Drehachse bewegt, so dass die Rolle den Schlauch nicht mehr vollständig okkludiert.

Auf diese Weise werden zu hohe Blutdrücke sicher vermieden. Tritt ein Betriebszustand auf, bei dem der Blutdruck stromabwärts der Schlauchrollenpumpe die Federkraft der Schlauchrollen, mit der der einliegende Schlauch an das Pumpbett gepresst wird, übersteigt, ist das mit einer stark ansteigenden Hämolyse verbunden.

Im Normalfall, also bei okkludierender Förderung, geschieht die Freisetzung von Hämoglobin bei Schlauchrollenpumpen weitgehend unabhängig vom Blutdruck proportional zur Pumprate.

Bei Blutbehandlungen und insbesondere bei Hämodialysebehandlungen ist der Blutfluss ein maßgeblicher Behandlungsparameter. Dieser wird in der Regel vom medizinischen Personal vorgegeben und als Sollwert über eine Bedienerschnittstelle in das Dialysegerät eingegeben. Dieses stellt entsprechend die Pumprate der Blutpumpe ein und hält die vorgegeben Blutflussrate weitgehend ohne Berücksichtigung des Blutdrucks an verschiedenen Stellen des extrakorporalen Blutkreislaufs aufrecht. Nur eine Überschreitung eines Grenzwerts für den Druck kann zu einer Unterbrechung oder einem Abbruch der Behandlung führen, wobei die Blutpumpe gestoppt werden kann, und der Bediener alarmiert werden kann.

Die Blutpumpe kann beispielsweise eine Schlauchrollenpumpe (nicht beanspruchte Ausführungsform) oder eine Zentrifugalpumpe (beanspruchte Ausführungsform) sein.

Bedingt durch die unterschiedlichen Charakteristika beider Pumpprinzipien gibt es bei Blutbehandlungsgeräten mit Zentrifugalpumpen zur Blutförderung Betriebs-parameter, für die die zu erwartende Blutschädigung geringer ist, als bei ansonsten vergleichbaren Blutbehandlungsgeräten, die mit Schlauchrollenpumpen zur Blutförderung ausgerüstet sind, wenn Zentrifugalpumpe und Schlauchrollenpumpe das Blut mit identischer Pumprate fördern.

Es kann andererseits in der therapeutischen Praxis aber vorkommen, dass die initial eingestellte Pumprate der Zentrifugalpumpe zu Betriebsparametern führt, bei denen die Verwendung von Zentrifugalpumpen zur Blutförderung gegenüber dem ansonsten vergleichbaren Blutbehandlungsgeräten, das mit einer Schlauchrollenpumpe zur Blutförderung ausgerüstet ist, im Hinblick auf die Blutschädigung nachteilig ist.

Eine Ausführungsform der Erfindung sieht daher vor, in einem solchen Fall die Pumprate der Zentrifugalpumpe so zu verändern, dass die zu erwartende Blutschädigungsrate zumindest geringer wird, bzw. der Betrieb im Vergleich mit einem Blutbehandlungsgerät, das mit einer Schlauchrollenpumpe zur Blutförderung ausgerüstet ist und das Blut mit identischer Pumprate fördert, nicht mehr nachteilig ist.

Eine weitere Ausführungsform der Erfindung sieht vor, bei Blutbehandlungsgeräten, die mit Schlauchrollenpumpen zur Blutförderung ausgerüstet sind, die initial eingestellte Pumprate im Falle, dass sich solche Betriebsparameter hierdurch ergeben, durch die der Blutdruck stromabwärts der Schlauchrollenpumpe so groß werden würde, dass die Okklusionskraft der Pumpe, also die Federkraft, mit der die Rollen der Schlauchrollenpumpe den einliegenden Schlauch gegen das Pumpbett pressen, nicht mehr ausreicht, um den Blutschlauch vollständig zu okkludieren, solange zu verringern, bis der Blutdruck stromabwärts der Schlauchrollenpumpe den der Okklusionskraft entsprechenden Grenzwert für den Blutdruck unterschreitet. Die Okklusionskraft ist hierbei ein die Schlauchrollenpumpe charakterisierender Pumpenparameter.

Ziel aller Ausführungsformen ist es, dass das Blut möglichst wenig geschädigt wird.

### Kurze Beschreibung der Figuren

Weitere Einzelheiten und Vorteile der Erfindung werden anhand von in den Zeichnungen dargestellten Ausführungsbeispielen näher beschrieben.

Es zeigen:
Die Figur 1 eine schematische Darstellung eines als Hämodialysegerät ausgeführten Blutbehandlungsgeräts im Einklang mit der Lehre der vorliegenden Erfindung;
die Figur 2 ein Diagramm, welches die Freisetzung von Hämoglobin bei der Förderung von Blut in Abhängigkeit von Betriebsparametern bei der Verwendung von Zentrifugalpumpen und Schlauchrollenpumpen zeigt im Einklang mit der Lehre der vorliegenden Erfindung;
die Figur 3 ein weiteres Diagramm, welches den Regelbereich für die Pumprate bei Zentrifugalpumpen zur Förderung von Blut darstellt im Einklang mit der Lehre der vorliegenden Erfindung;
die Figur 4 eine vereinfachte schematische Darstellung eines Blutbehandlungsgeräts im Einklang mit der Lehre der vorliegenden Erfindung;
die Figur 5 ein Ablaufdiagramm für ein beispielhaftes Verfahren zur Regelung der Pumprate von Pumpen zur Förderung von Blut im Einklang mit der Lehre der vorliegenden Erfindung und
die Figur 6 ein Ablaufdiagramm für ein beispielhaftes Verfahren zur Vorgabe der Pumprate von Pumpen zur Förderung von Blut im Einklang mit der Lehre der vorliegenden Erfindung.

### Ausführliche Beschreibung der Figuren

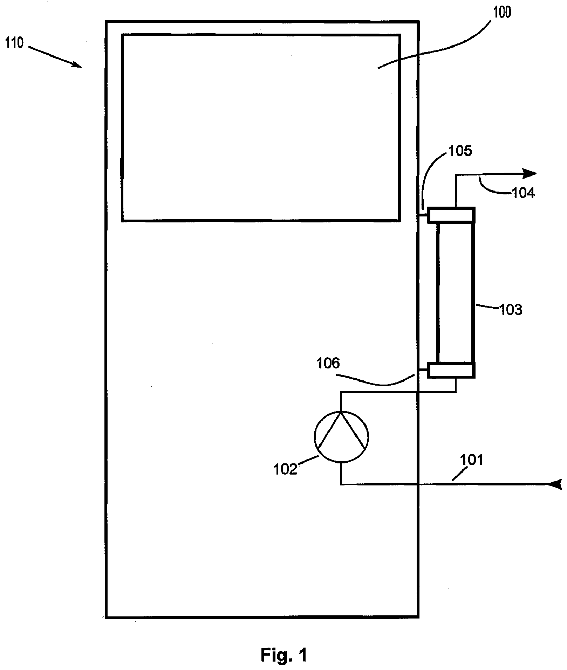

In Figur 1 ist schematisch ein als Hämodialysegerät ausgeführtes Blutbehandlungsgeräts dargestellt. Das Hämodialysegerät 110 umfasst Teile eines extrakorporalen Blutkreislaufs mit einer arteriellen Blutleitung 101, die Blut eines Patienten (nicht dargestellt) ableitet. Die Blutpumpe 102 fördert das Blut über den in sie eingelegten Schlauchabschnitt durch einen Dialysator 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Die verwendeten Leitungen und Schlauchabschnitte, insbesondere auch die Teile, die den extrakorporalen Blutkreislauf ausbilden, sind in der Regel Einmalteile, die nach Gebrauch verworfen werden. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das Blut des Patienten kann so entwässert werden. Das Dialysat wird in dem Hämodialysegerät 110 hergestellt und nach Gebrauch verworfen. Die Blutpumpe 103 kann im Einklang mit der Lehre der vorliegenden Erfindung als Zentrifugalpumpe (beanspruchte Ausführungsform) oder als Schlauchrollenpumpe (nicht beanspruchte Ausführungsform) ausgeführt sein.

Zentrifugalpumpen können insbesondere bei Hämodialysegeräten als Blutpumpe Anwendung finden, wenn das Flügelrad der Zentrifugalpumpe in einer als Einmalteil ausgeführten Kassette angeordnet ist, die weitere Teile des extrakorporalen Blutkreislaufs fluiddicht beinhalten kann, wie beispielsweise Blutleitungen, Gerinselfänger und Tropfkammern. Das angetriebene Flügelrad wird hierbei zusammen mit der Kassette nach Gebrauch verworfen. Die das Flügelrad antreibenden Mittel sind dabei in dem Hämodialysegerät untergebracht und treiben das Flügelrad über eine magnetische Kopplung an.

Das Hämodialysegerät 110 ist mit einem in Figur 1 nicht dargestellten Steuergerät, das die Blutpumpe ansteuern bzw. regeln kann sowie mit Sensoren zur Aufnahme von Blutdrücken im extrakorporalen Blutkreislauf ausgerüstet. Das Steuergerät ist dabei so konfiguriert, dass es die beschriebenen Verfahren ausführen kann.

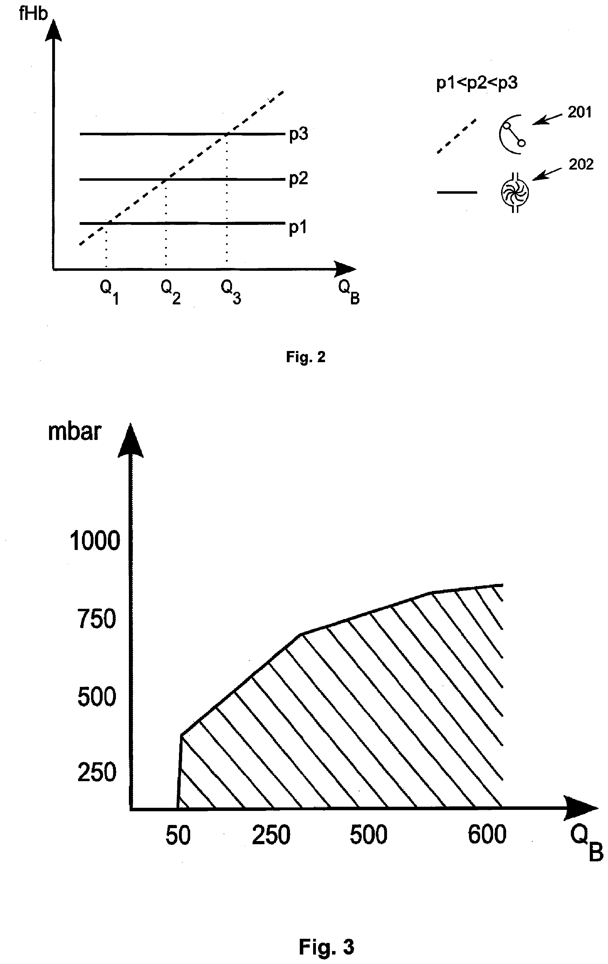

Die Figur 2 zeigt ein Diagramm, das schematisch die Blutschädigung für zwei Blutbehandlungsgeräte darstellt, von denen eines zur Förderung des Blutes eine Zentrifugalpumpe verwendet und das andere für den gleichen Zweck eine Schlauchrollenpumpe, die ansonsten aber identisch sind.

Hierbei kennzeichnet die gestrichelte Linie das Blutbehandlungsgerät mit der Schlauchrollenpumpe, symbolisiert durch die Darstellung 201. Die durchgezogenen Linien gelten für das Blutbehandlungsgerät mit der Zentrifugalpumpe, symbolisiert durch die Darstellung 202.

Auf der Abszisse ist der Blutfluss Q_{B} aufgetragen, der Ordinatenwert kennzeichnet die Entstehung freien Hämoglobins (fHb).

Die Entstehung des freien Hämoglobins ist für die Schlauchrollenpumpe weitgehend unabhängig vom Blutdruck, während sie bei der Zentrifugalpumpe mit dem Druck zusammenhängt (in Figur 2 beispielhaft für drei unterschiedliche Drücke p1, p2, p3 dargestellt).

Die Figur 2 zeigt, dass die Blutschädigung bei der Zentrifugalpumpe mit größer werdendem Blutdruck stromabwärts zunimmt, vom Blutfluss durch die Zentrifugalpumpe aber nicht wesentlich beeinflusst wird.

Bei Verwendung einer Schlauchrollenpumpe hingegen ist die Blutschädigung im Normalbetrieb, also mit okkludierenden Pumpenrollen, vom Blutdruck stromabwärts im Wesentlichen unabhängig, während sie mit zunehmendem Blutfluss steigt.

Somit ergibt sich abhängig vom jeweils gerade vorherrschenden Blutdruck p stromabwärts der Pumpe für den Betrieb des Blutbehandlungsgeräts mit der Zentrifugalpumpe ein bevorzugter Bereich für den Blutfluss Q_{B}, bei dem die Blutschädigung kleiner ausfällt, als bei einem Vergleichsgerät mit einer Schlauchrollenpumpe und identischem Blutdruck p stromabwärts der Schlauchrollenpumpe. Dieser liegt im Beispiel von Figur 2 bei einem Druck p1 zwischen Q1 und dem maximal zulässigen Blutfluss, bei einem Druck p2 zwischen Q2 und dem maximal zulässigen Blutfluss und bei einem Druck p3 zwischen Q3 und dem maximal zulässigen Blutfluss.

Im Einklang mit der Lehre der vorliegenden Erfindung kann nun während des Betriebes eines Blutbehandlungsgerätes, das zur Förderung des Blutes eine Zentrifugalpumpe verwendet, geprüft werden, ob der Blutfluss unter Berücksichtigung der gerade vorherrschenden Blutdrücke im extrakorporalen Kreislauf in diesem Bereich liegt.

Sollte dies nicht der Fall sein, liegt der Arbeitspunkt der Zentrifugalpumpe, definiert durch die Betriebsparameter Pumprate und Blutdruck stromaufwärts oder stromabwärts der Blutpumpe, oder Differenz dieser Blutdrücke, analog Figur 2 auf der linken Seite der gestrichelten Linie. Die Steuereinheit kann dann den Blutfluss solange verändern, bis er zumindest auf der gestrichelten Linie liegt.

Bei der Veränderung der Pumprate ändert sich durch den Zusammenhang zwischen Flussrate, Flusswiderstand und Flüssigkeitsdruck auch der Blutdruck stromauf- und abwärts der Zentrifugalpumpe. Bezug nehmend auf die Figur 2 bedeutet eine solche Anpassung, dass sich der Arbeitspunkt der Zentrifugalpumpe nicht auf einer horizontalen Linie ändert. Demnach kann sowohl eine Erhöhung der Pumprate, als auch eine Erniedrigung der Pumprate dazu führen, dass sich der Arbeitspunkt der Zentrifugalpumpe wieder im bevorzugten Bereich einstellt, nämlich in Figur 2 auf der gestrichelten Linie oder rechts davon.

In der Praxis zeigt sich, dass der Druck stromabwärts der Zentrifugalpumpe aufgrund von Engstellen im Strömungspfad stromabwärts der Zentrifugalpumpe, beispielsweise an Kanülen oder Kathetern, quadratisch mit der Erhöhung der Pumprate steigt. Deshalb ist die bevorzugte Veränderung der Pumprate eine Erniedrigung. In besonderen Fällen, beispielsweise wenn der Arbeitspunkt nur wenig links der gestrichelten Linie in der Figur 2 liegt, oder wenn besondere Schlauchsets zur Blutförderung benutzt werden, kann auch eine Erhöhung dazu führen, dass sich der Arbeitspunkt der Zentrifugalpumpe wieder im bevorzugten Bereich einstellt, nämlich in Figur 2 auf der gestrichelten Linie oder rechts davon.

In einer Ausführungsform kann die Steuereinheit auch in einem iterativen Prozess eine neue Pumprate einstellen, indem die Veränderung der Pumprate in positiver oder negativer Richtung nur in kleinen Schritten, beispielsweise in 1% Schritten erfolgt, und nach jedem Schritt geprüft wird, ob die Veränderung zu einer Verkleinerung des Abstands des Arbeitspunktes der Zentrifugalpumpe von der gestrichelten Linie in der Figur 2 geführt hat.

Vorstellbar ist auch, dass die Steuereinheit die Veränderung der Pumprate innerhalb therapeutisch erlaubter Grenzen sowohl als Erhöhung als auch Erniedrigung in diesem iterativen Prozess durchführt und so die bestmögliche neue Pumprate bestimmt, in dem für jede Blutpumpenrate innerhalb des erlaubten Bereichs die Blutschädigungsrate protokolliert wird, beispielsweise durch Abspeichern in einem Datenspeicher, und anschließend jene Pumprate mit der niedrigsten protokollierten Blutschädigung eingestellt wird. Es ist möglich, dass sich innerhalb der therapeutisch erlaubten Grenzen für die Blutpumpenrate kein Arbeitspunkt einstellt, der rechts der gestrichelten Linie liegt. In diesem Fall wird im Einklang mit der vorliegenden Erfindung die Pumpenrate mit der niedrigsten verbundenen Blutschädigungsrate ermittelt, bzw. eingestellt.

Es wird demnach also überprüft, ob ein Betriebsparameter der Blutpumpe einen Grenzwert verletzt. Dieser Grenzwert ist abhängig vom zuvor bestimmten Blutdruck. Dabei ist es unerheblich, ob der Blutdruck stromaufwärts des Blutdrucks oder stromabwärts des Blutdrucks gemessen wird, oder dem Steuergerät bekannt gemacht wird, denn bei Kenntnis der Drehzahl der Zentrifugalpumpe kann der jeweils fehlende Flüssigkeitsdruck aus einer hinterlegten Kennlinie der Pumpe bestimmt werden.

Dieser Grenzwert, dem die Schnittstelle zwischen durchgezogener Linie und gestrichelter Linie in Figur 2 entspricht, ist in diesem Fall eine vom gerade vorherrschenden Blutdruck p stromabwärts der Zentrifugalpumpe abhängiger Blutfluss, also die Pumprate der Zentrifugalpumpe.

Eine Verletzung dieses Grenzwertes bedeutet, dass die Zentrifugalpumpe mit einer nachteiligen Pumprate fördert und dementsprechend mehr Blutschädigung verursacht als eine in einem Vergleichsgerät eingebaute Schlauchrollenpumpe mit identischer Pumprate. Das Steuergerät des Blutbehandlungsgeräts wird folglich die Pumprate der Zentrifugalpumpe soweit verändern und anpassen, bis die Pumprate wie weiter oben dargestellt nicht mehr nachteilig ist.

Da diese angepasste Pumprate eine andere ist, als die initial am Blutbehandlungsgerät eingestellte Pumprate, kann zuvor überprüft werden, ob die angepasste Pumprate im Einklang mit der Therapie des Patienten erlaubt ist.

Es ist vorstellbar, dass bevor die Blutbehandlungsmaschine die Pumprate verändert, eine Meldung an das behandelnde medizinische Personal ergeht. Beispielsweise kann eine entsprechende Meldung auf einem Bildschirm angezeigt werden, die vom medizinischen Personal durch Benutzereingaben quittiert werden muss. Weiterhin ist vorstellbar, dass die Maschine einen Bereich von Blutflüssen anzeigt, für den die zu erwartende Blutschädigung geringer ausfällt, als bei einem Vergleichsgerät mit Schlauchrollenpumpe und das medizinische Personal nach Abwägung der Therapiefolgen einen konkreten Blutfluss auswählt.

Die Ermittlung der Kurven aus Figur 2 kann beispielsweise in Laborversuchen erfolgen, bei dem Vergleichsgeräte mit Zentrifugalpumpen und Schlauchrollen-pumpen unter variierenden Bedingungen bezüglich Blutdruck, Blutfluss und daraus resultierender Blutschädigung miteinander verglichen werden.

Derart gewonnene Vergleichsdaten können im Blutbehandlungsgerät abgespeichert werden, entweder in Form einer Tabelle, oder in Form von aus den Versuchsdaten gewonnenen mathematischen Beschreibungen. Unabhängig davon, wie die Daten hinterlegt sind, wichtig ist nur, dass im medizinischen Behandlungsgerät die Beziehung zwischen Blutdruck stromabwärts der Zentrifugalpumpe, oder dem Differenzdruck zwischen Pumpenein und -ausgang bzw. der Drehzahl der Pumpe, und Blutflüssen hinterlegt ist.

Die Figur 3 zeigt ein beispielhaftes Diagramm, das für eine als Zentrifugalpumpe ausgeführte Blutpumpe einen bevorzugten, in Figur 3 schraffierten Bereich für den Arbeitspunkt der Zentrifugalpumpe anzeigt. Die Knickstellen der oberen Begrenzung des schraffierten Arbeitsbereichs entsprechen hierbei den Schnittpunkten der gestrichelten Linie mit den durchgezogenen Linien in Figur 2. In diesem Bereich sind der Differenzdruck aus stromabwärtigen und stromaufwärtigen Blutdruck, der an der Ordinate aufgetragen ist, und der Blutfluss in einem derartigen Verhältnis, dass eine Veränderung der Pumprate nicht notwendig ist.

Stellt sich ein Arbeitspunkt außerhalb des schraffierten Bereichs ein, greift die Regelung im Einklang mit der Lehre der vorliegenden Erfindung derart ein, dass die Pumprate der Zentrifugalpumpe solange verändert wird, bis der Arbeitspunkt wieder im schraffierten Bereich liegt.

Der in der Figur 3 beispielhaft gezeigte schraffierte Bereich kann abhängig von der Ausführungsform der zugrunde liegenden Blutpumpe einen anderen Bereich abdecken.

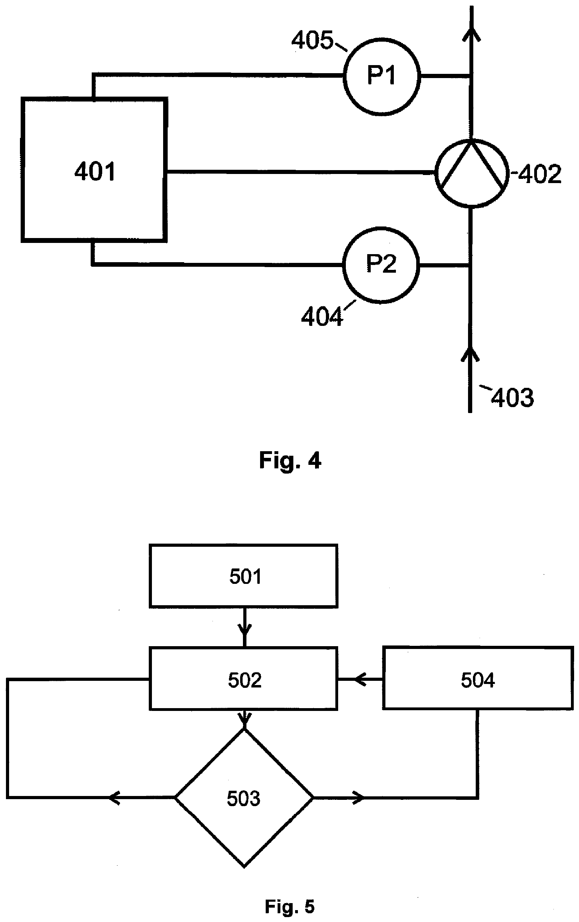

Die Figur 4 zeigt eine vereinfachte schematische Darstellung eines Blutbehandlungsgeräts, welches zur Vorgabe einer Pumprate einer Blutpumpe konfiguriert ist, im Einklang mit der Lehre der vorliegenden Erfindung. Dieses umfasst eine Steuergerät 401, beispielsweise als Mikrokontroller ausgeführt, welches die Pumprate der Blutpumpe 402 ansteuern kann. Dem Steuergerät können die Messwerte der Drucksensoren 404 und/oder 405 stromaufwärts der Blutpumpe 402 (P1) und/oder stromabwärts der Blutpumpe 402 (P2) zugeführt werden. Die Blutpumpe fördert Blut innerhalb einer Blutleitung 403, die Teil eines extrakorporalen Blutkreislaufs sein kann, beispielsweise während einer Hämodialysebehandlung.

Das Steuergerät 401 ist im Einklang mit der Lehre der vorliegenden Erfindung derart konfiguriert, eine dem Steuergerät beispielsweise durch Eingabe eines Bedieners in eine Bedienerschnittstelle, die beispielsweise als Touchscreen ausgeführt sein kann, zuvor bekannt gemachte Pumprate der Blutpumpe durch entsprechende Steuersignale an die Blutpumpe 402 einzustellen und im Falle, dass der Blutdruck P2 stromaufwärts und/oder der Blutdruck P1 stromabwärts der Blutpumpe und/oder die Differenz der Blutdrücke stromaufwärts und stromabwärts der Blutpumpe einen Grenzwert verletzt, der von einem Betriebsparameter der Blutpumpe abhängt, die Pumprate der Blutpumpe solange zu verändern, bis der Grenzwert nicht mehr überschritten wird.

Eine Verletzung des Grenzwerts kann vorliegen, wenn der Differenzdruck P1-P2 analog der Figur 3 nicht im schraffierten Bereich liegt, wobei der Grenzwert von der Pumprate als Betriebsparameter abhängt und wobei die Blutpumpe als Zentrifugalpumpe ausgeführt ist. Als Reaktion kann das Steuergerät die Pumprate der Zentrifugalpumpe iterativ solange verändern, bis der aus den Druckwerten P1 und P2 bestimmte Differenzdruck P1-P2 wieder im schraffierten Bereich liegt.

Eine Verletzung des Grenzwerts kann auch vorliegen, wenn der Druck P2 stromaufwärts der Pumpe, die als Schlauchrollenpumpe oder Zentrifugalpumpe ausgeführt sein kann, niedriger als ein Grenzwert ist. Es kann nämlich ab einem bestimmten kritischen Unterdruck im Blut, beispielsweise 400mbar unterhalb des Umgebungsdruckes, zum gehäuften Auftreten von Mikroluftbläschen im Blut kommen. Um dies zu verhindern, kann das Steuergerät nach Feststellen einer Unterschreitung des Druckes P2 unterhalb eines Grenzwertes die Pumprate der Pumpe sukzessive reduzieren, bis der Druck P2 wieder oberhalb dieses Grenzwertes liegt. Eine Reduzierung des Blutflusses wird zugunsten höherer Patientensicherheit demnach in Kauf genommen. Ebenso wird gegebenenfalls die Anzahl der aufgrund auftretender Mikrobläschen ausgelöster Alarme reduziert.

Eine weitere Verletzung des Grenzwertes kann vorliegen, wenn der Blutdruck im Falle, dass die Blutpumpe als Zentrifugalpumpe ausgeführt ist, unterhalb eines Grenzwertes liegt, der von der Pumprate als Betriebsparameter der Blutpumpe abhängt, analog der Figur 2. In diesem Fall ist die zu erwartende Blutschädigung durch die Zentrifugalpumpe größer als eine zum Vergleich herangezogene Schlauchrollenpumpe mit identischer Pumprate. Das Steuergerät erhöht in diesem Fall die Pumprate der Zentrifugalpumpe solange, bis der gemessene Druck stromabwärts der Blutpumpe zumindest dem Grenzwert entspricht. Dieser Grenzwert entspricht der gestrichelten Linie des in Figur 2 dargestellten Diagramms.

Eine weitere Verletzung des Grenzwertes kann vorliegen, wenn der Blutdruck stromabwärts der Blutpumpe im Falle, dass die Blutpumpe eine Schlauchrollenpumpe ist, oberhalb eines Grenzwertes liegt, der mit der Federkraft der federnd gelagerten Pumprollen (Okklusionskraft) als Betriebsparameter korrespondiert. Dieser Grenzwert ist abhängig von den Eigenschaften des im Pumpbett einliegenden Schlauches, wie beispielsweise Elastizität und Durchmesser, und kann dem Steuergerät in beliebiger Weise bekannt gemacht werden, beispielsweise durch eine Bedienereingabe des Schlauchtyps, für den entsprechende Grenzwerte in einem Datenspeicher, auf den das Steuergerät zugreifen kann, abgespeichert sind. Das Steuergerät reduziert in Falle einer Verletzung des Grenzwertes die Pumprate der Schlauchrollenpumpe solange, bis der gemessene Druck stromabwärts der Blutpumpe den Grenzwert nicht mehr verletzt.

Die Figur 5 zeigt ein Ablaufdiagramm für ein beispielhaftes Verfahren zur Vorgabe der Pumprate von Pumpen zur Förderung von Blut im Einklang mit der Lehre der vorliegenden Erfindung, wenn das Verfahren auf einer Vorrichtung nach der Figur 4 abläuft.

Im Schritt 501 wird eine initiale Pumprate für eine Blutpumpe eingestellt oder eine zuvor eingestellte Pumprate für diese Blutpumpe ermittelt. Im Schritt 502 wird der Blutdruck stromabwärts und/oder stromaufwärts der Blutpumpe ermittelt. Der so ermittelte Wert, oder ein aus diesen gemessenen Daten gebildeter Wert, beispielsweise der Differenzdruck aus den Blutdrücken stromabwärts und stromaufwärts der Blutpumpe, wird im Schritt 503 mit einem Grenzwert verglichen. Der Grenzwert hängt in schon beschriebener Weise von zumindest einem Betriebsparameter oder Pumpenparameter der Blutpumpe ab, beispielsweise mit der aktuellen Pumprate oder der Okklusionskraft für die Blutpumpe.

Wird der Grenzwert von dem so ermittelten Wert verletzt, erfolgt im Schritt 504 eine Veränderung der Pumprate. Dies kann eine Erhöhung oder eine Erniedrigung der Pumprate bedeuten. Diese Änderung der Pumprate kann in einer (iterativen) Regelschleife bestehend aus den Schritten 502, 503 und 504 erfolgen, bis der Grenzwert nicht mehr verletzt wird, wobei bei jeder Änderung der Pumpenrate im Schritt 503 überprüft wird, ob der Grenzwert verletzt wird.

Wird der Grenzwert nicht verletzt, wird die Pumprate nicht verändert. Dennoch wird in den Schritten 502 und 503 weiterhin permanent überwacht, ob der Grenzwert verletzt wird.

Vorstellbar ist auch, dass das Verfahren nach Figur 5 derart durchgeführt wird, dass wenn eine im Schritt 501 initial eingestellte oder ermittelte Pumprate für die Blutpumpe zu einer Verletzung des Grenzwertes, festgestellt im Schritt 503, führt, im Schritt 504 der gesamte therapeutisch erlaubte Bereich für die Pumprate der Blutpumpe in kleinen Schritten durchfahren wird, und für jede erlaubte Pumprate die Blutschädigung, gekennzeichnet durch den Arbeitspunkt analog der Figur 2, der durch die bekannte Pumprate und die Blutdrücke stromabwärts und/oder stromaufwärts der Blutpumpe im Schritt 502 bestimmt wird, protokolliert wird. Hierzu werden die so bestimmten Arbeitspunkte für jede erlaubte Pumprate in einem Datenspeicher abgelegt und anschließend jene Pumprate eingestellt bzw. vorgegeben, für die die sich einstellenden Blutschädigungsrate (also fHb in Figur 2) am geringsten ist.

Eine so ermittelte neue Pumprate, für die die sich einstellende Blutschädigungsrate am geringsten ist, muss nicht zwingend mit einem Arbeitspunkt der Blutpumpe verbunden sein, der im bevorzugten Bereich analog der Figur 3 liegt. In diesem Fall ist der Abstand zum erlaubten Bereich aber möglichst klein oder minimal.

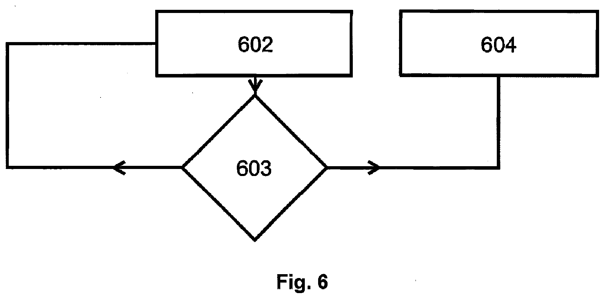

Die Figur 6 zeigt ein Ablaufdiagramm für ein beispielhaftes Verfahren zur Vorgabe der Pumprate von Pumpen zur Förderung von Blut im Einklang mit der Lehre der vorliegenden Erfindung.

Im Schritt 602 wird der Blutdruck stromabwärts und/oder stromaufwärts der Blutpumpe ermittelt.

Der so ermittelte Wert, oder ein aus diesen gemessenen Daten gebildeter Wert, beispielsweise der Differenzdruck aus den Blutdrücken stromabwärts und stromaufwärts der Blutpumpe, wird im Schritt 603 mit einem Grenzwert verglichen. Der Grenzwert hängt in schon beschriebener Weise von zumindest einem Betriebsparameter oder Pumpenparameter der Blutpumpe ab.

Wird der Grenzwert von dem so ermittelten Wert verletzt, erfolgt im Schritt 604 die Vorgabe einer neuen Pumprate derart, dass der Grenzwert nicht mehr verletzt werden würde, oder die Differenz des Blutdrucks stromabwärts und/oder stromaufwärts der Blutpumpe von diesem Grenzwert kleiner werden würde.

Wird der Grenzwert nicht verletzt, erfolgt keine Vorgabe einer neuen Pumprate. Dennoch wird in den Schritten 602 und 603 weiterhin permanent überwacht, ob der Grenzwert verletzt wird.

## Patentansprüche

1. Vorrichtung umfassend ein Steuergerät, eine als Zentrifugalpumpe ausgeführte Blutpumpe, welche durch das Steuergerät ansteuerbar ist und zumindest einen Sensor zur Erfassung des Blutdrucks stromaufwärts und/oder stromabwärts der Blutpumpe, wobei das Steuergerät so konfiguriert ist, eine initiale Pumprate der Blutpumpe einzustellen oder zu ermitteln und im Falle, dass der Blutdruck stromaufwärts und/oder stromabwärts der Blutpumpe und/oder die Differenz der Blutdrücke stromaufwärts und stromabwärts der Blutpumpe einen Grenzwert verletzt, der von der Pumprate der Blutpumpe abhängt und wobei der Grenzwert mit einer Blutschädigungsrate verknüpft ist, die Pumprate der Blutpumpe solange zu verändern, bis der Grenzwert nicht mehr verletzt wird, oder die Differenz des Blutdrucks stromabwärts der Blutpumpe und diesem Grenzwert oder stromaufwärts der Blutpumpe und diesem Grenzwert kleiner wird.

2. Vorrichtung nach Anspruch 1, wobei das Steuergerät dazu eingerichtet ist, den Blutdruck stromaufwärts der Zentrifugalpumpe aus dem Blutdruck stromabwärts der Zentrifugalpumpe und der Drehzahl der Zentrifugalpumpe zu bestimmen, oder wobei das Steuergerät dazu eingerichtet ist, den Blutdruck stromabwärts der Zentrifugalpumpe aus dem Blutdruck stromaufwärts der Zentrifugalpumpe und der Drehzahl der Zentrifugalpumpe zu bestimmen.

3. Vorrichtung nach einem der vorhergehenden Ansprüche, wobei ein bevorzugter Bereich für einen Arbeitspunkt der Blutpumpe, der durch einen von der Pumprate abhängigen Maximalblutdruck stromabwärts und/oder stromaufwärts der Zentrifugalpumpe definiert ist, hinterlegt ist, und wobei das Einstellen einer neuen Pumprate derart erfolgt, dass der Arbeitspunkt der Blutpumpe innerhalb des erlaubten Bereichs liegt, oder sich ein möglichst kleiner Abstand zwischen Arbeitspunkt und erlaubtem Bereich einstellt.

4. Blutbehandlungsgerät, insbesondere ein Dialysegerät, ein Gerät zur Unterstützung der Herz-Lungentätigkeit oder ein Gerät zur Unterstützung der Leberfunktion mit einer Vorrichtung nach einem vorhergehenden Ansprüche.

5. Blutbehandlungsgerät nach Anspruch 4 mit einem extrakorporalen Blutkreislauf, umfassend eine Blutbehandlungskomponente, insbesondere als Dialysator ausgebildet und einem arteriellen und einem venösen Blutzweig.

6. Blutbehandlungsgerät nach Anspruch 5, wobei zumindest Teile des extrakorporalen Blutkreislaufes als Einmalteil ausgebildet sind.

## Claims

1. Apparatus comprising a controller, a blood pump which is designed as a centrifugal pump and controllable by the controller, and at least one sensor for measuring the blood pressure upstream and/or downstream of the blood pump, wherein the controller is configured to set or ascertain an initial pump rate of the blood pump and, should the blood pressure upstream and/or downstream of the blood pump and/or the difference between the blood pressures upstream and downstream of the blood pump breach a limit value which depends on the pump rate of the blood pump and which is linked to a blood damaging rate, change the pump rate of the blood pump until the limit value is no longer breached, or the difference between the blood pressure downstream of the blood pump and this limit value or upstream of the blood pump and this limit value becomes smaller.

2. Apparatus according to Claim 1, wherein the controller is configured to determine the blood pressure upstream of the centrifugal pump from the blood pressure downstream of the centrifugal pump and the rotational speed of the centrifugal pump or wherein the controller is configured to determine the blood pressure downstream of the centrifugal pump from the blood pressure upstream of the centrifugal pump and the rotational speed of the centrifugal pump.

3. Apparatus according to any of the preceding claims, wherein a preferred range for an operating point of the blood pump, which is defined by a pump rate-dependent maximum blood pressure downstream and/or upstream of the centrifugal pump, is stored and wherein a new pump rate is set in such a way that the operating point of the blood pump is within the allowed range or that a distance that is as small as possible sets in between the operating point and the allowed range.

4. Blood treatment device, in particular a dialysis device, a device for assisting the cardiopulmonary activity or a device for assisting the liver function, comprising an apparatus according to any of the preceding claims.

5. Blood treatment device according to Claim 4 having an extracorporeal blood circuit, comprising a blood treatment component, embodied as a dialyzer in particular, and an arterial and a venous blood branch.

6. Blood treatment device according to Claim 5, wherein at least parts of the extracorporeal blood circuit are designed as single use parts.

## Revendications

1. Dispositif comprenant une unité de commande, une pompe à sang qui est conçue comme une pompe centrifuge et qui peut être commandée par l'unité de commande, et au moins un capteur destiné à détecter la pression sanguine en amont et/ou en aval de la pompe à sang, l'unité de commande étant conçue pour régler ou déterminer un débit de pompage initial de la pompe à sang et, dans le cas où la pression sanguine en amont et/ou en aval de la pompe à sang et/ou la différence des pressions sanguines en amont et en aval de la pompe à sang franchit une valeur limite qui dépend du débit de pompage de la pompe à sang et la valeur limite étant liée à un taux de lésions sanguines, pour modifier le débit de pompage de la pompe à sang jusqu'à ce que la valeur limite ne soit plus franchie, ou la différence entre la pression sanguine en aval de la pompe à sang et cette valeur limite ou en amont de la pompe à sang et cette valeur limite devienne plus petite.

2. Dispositif selon la revendication 1, l'unité de commande étant conçue pour déterminer la pression sanguine en amont de la pompe centrifuge à partir de la pression sanguine en aval de la pompe centrifuge et de la vitesse de rotation de la pompe centrifuge, ou l'unité de commande étant conçue pour déterminer la pression sanguine en aval de la pompe centrifuge à partir de la pression sanguine en amont de la pompe centrifuge et de la vitesse de rotation de la pompe centrifuge.

3. Dispositif selon l'une des revendications précédentes, une zone préférée d'un point de fonctionnement de la pompe à sang, qui est définie par une pression sanguine maximale dépendant du débit de pompage en aval et/ou en amont de la pompe centrifuge, étant stockée, et un nouveau débit de pompage étant réglé de manière à ce que le point de fonctionnement de la pompe à sang se trouve à l'intérieur de la zone autorisée, ou la distance la plus petite possible entre le point de fonctionnement et la zone autorisée soit atteinte.

4. Unité de traitement de sang, en particulier unité de dialyse, unité d'assistance à l'activité cardio-pulmonaire ou unité d'assistance à la fonction hépatique, comprenant un dispositif selon l'une des revendications précédentes.

5. Unité de traitement de sang selon la revendication 4 à circulation sanguine extracorporelle, ladite unité comprenant un élément de traitement de sang, notamment conçu comme un dialyseur et une branche de sang artériel et une branche de sang veineux.

6. Unité de traitement de sang selon la revendication 5, au moins des parties de la circulation sanguine extracorporelle étant conçues comme des pièces à usage unique.
